# EUROPEAN PATENT SPECIFICATION

(11) **EP 2 972 514 B1**
(45) Date of publication and mention of the grant of the patent: **23.12.2020**
(21) Application number: 14710950.8
(22) Date of filing: 12.03.2014
(51) Int. Cl.: G01R 33/02, A61B 90/00

(54) **MAGNETIC DETECTOR**
MAGNETDETEKTOR
DÉTECTEUR MAGNÉTIQUE

(30) Priority: 13.03.2013 US 201313799334; 13.03.2013 US 201313799480
(43) Date of publication of application: 20.01.2016
(73) Proprietor: Endomagnetics Ltd., Cowley Road Cambridge CB4 0WS (GB)
(72) Inventor: HATTERSLEY, Simon Richard, Bickley Kent BR1 2DF (GB); LAITENBERGER, Peter Georg, Cambridge Cambridgeshire CB4 3DN (GB); BRAZDEIKIS, Audrius, Missouri City, Texas 77459 (US)
(74) Representative: Boult Wade Tennant LLP
(86) International application number: PCT/GB2014/050732
(87) International publication number: WO 2014/140567

(56) References cited:
- WO-A1-98/07052
- DE-U1- 29 724 862
- DE-U1- 29 724 862
- US-A- 2 614 164
- US-A- 5 005 001
- US-A1- 2009 164 161
- US-A1- 2009 164 161
- US-A1- 2011 133 730
- US-B1- 6 304 075
- WILLIAMSON S J ET AL: "Biomagnetism", JOURNAL OF MAGNETISM AND MAGNETIC MATERIALS, ELSEVIER, AMSTERDAM, NL, vol. 22, no. 2, 1 January 1981 (1981-01-01), pages 129-201, XP024463592, ISSN: 0304-8853, DOI: 10.1016/0304-8853(81)90078-0 [retrieved on 1981-01-01]

## Description

### FIELD OF THE INVENTION

The invention relates generally to the field of medical devices for locating tissue in preparation for surgery and more specifically for detecting magnetic markers in tissue for excision.

### BACKGROUND

The recent use of magnetic sensor probes to detect magnetic nanoparticles in the localization of sentinel lymph nodes in the staging of cancer in preparation for surgery has made the job of determining the location of the sentinel nodes easier for the surgeon. Further, the use of a probe to detect magnetic markers has also made relocating biopsy sites easier after pathology microscopic examination of excised tissue.

The inventors of the sensor probes for these systems seek to improve the design by: reducing thermal effects which cause coils in the sensor to shift with respect to one another and reduce the ability of the user to detect the signal from the magnetic nanoparticles; reducing interference caused by diamagnetic responses due to the body itself; and reducing the interference caused by eddy currents induced in objects near the sensor probe. In addition, it is desired that all these functional improvements be accomplished using a smaller sized probe with heightened sensitivity.

DE 29724862U1 describes an inductive proximity switch. US 2011/0133730 discusses a magnetic probe for use in locating magnetic material. US 2009/0164161 relates to a method to quantitatively measure an amount of bio-molecules in a sample, including providing a solution having magnetic nanoparticles. WO98/07052 (A1) is also pertinent for understanding the background of the invention. Williamson, S.J. et al., J. Magnetism and Magnetic Materials, vol. 22, January 1981, describes the study of magnetic fields originating in biological systems.

### SUMMARY OF THE INVENTION

The invention provides a magnetic susceptometer for locating tissue in preparation for excision by surgery as set out in claim 1. Embodiments provide a probe including a probe core having a first end and a second end, the probe core defining two regions for containing coils of wire, one of the regions being adjacent the first end of the cylindrical probe core; two sense coils, one each of the sense coils being located in a respective one of the regions; and two drive coils, one each of the drive coils being located in a respective one of the regions, wherein the regions are separated by a distance equal to or greater than the diameter of one of the coils and a source of a secondary magnetic drive field. The source of a secondary magnetic drive field may be a coil located on the probe. The frequency of the drive signal of the secondary magnetic drive field may be less than the frequency of the drive signal of the primary drive coils. In another embodiment, the source of the secondary magnetic drive field may be a rotating magnet being driven by a motor located in the probe. In yet another embodiment, the source of a secondary magnetic drive field may be a coil located at a distance from the probe.

The probe core may comprise a material with a thermal diffusivity of substantially ≥20 x 10⁻⁶ m²/s and a thermal expansion coefficient of substantially < 3 x 10⁻⁵/°C. In other embodiments, the probe core may comprise a material that has a thermal diffusivity of substantially ≥ 50 x 10⁻⁶ m²/s and a thermal expansion coefficient of substantially < 5 x 10⁻⁶/°C.

The invention also provides a method as set out in claim 11. In one embodiment, the method includes generating a drive frequency f₀; generating a secondary magnetic drive frequency f₁; and measuring the amplitude of a side lobe. In another embodiment, the side lobe is measured at a frequency equal to f₀ ± 2f₁.

In another embodiment, the method including generating a drive frequency f₀ generating a secondary magnetic drive frequency f₁; and measuring the ratio of the amplitude of a side lobe to the amplitude of the fundamental A_{f0}. In another embodiment, the amplitude (A_{f0±2f1}) of the side lobe is measured at a frequency equal to f₀±2f₁.

### BRIEF DESCRIPTION OF THE DRAWINGS

The structure and function of the invention can be best understood from the description herein in conjunction with the accompanying figures. The figures are not necessarily to scale, emphasis instead generally being placed upon illustrative principles. The figures are to be considered illustrative in all aspects and are not intended to limit the invention, the scope of which is defined only by the claims.
Fig. 1 is a block diagram of an embodiment of a probe constructed in accordance with the invention;
Fig. 2 is a block diagram of another embodiment of a probe constructed in accordance with the invention;
Fig. 3 is a cross-sectional diagram of the embodiment of the probe of Fig. 2;
Fig. 4 is a block diagram of a probe using a solenoid coil;
Figs. 5 (a-d) are cross-sections of four solenoid coil configurations;
Fig. 6a is a cross section of a solenoidal coil with additional coils on each end;
Fig. 6b is a graph of the field generated by the solenoidal coil of Fig. 6a;
Fig. 7 is a cross-sectional representation of a Helmholtz coil embodiment;
Fig. 8 is a graph of the modulation of a primary magnetic field with a secondary magnetic field and the resulting frequency spectrum;
Fig. 9 illustrates a secondary field generator for use in creating the secondary magnetic field shown in Fig. 8;
Fig. 10 illustrates a secondary field generator for use in creating the secondary magnetic field shown in Fig. 8, and

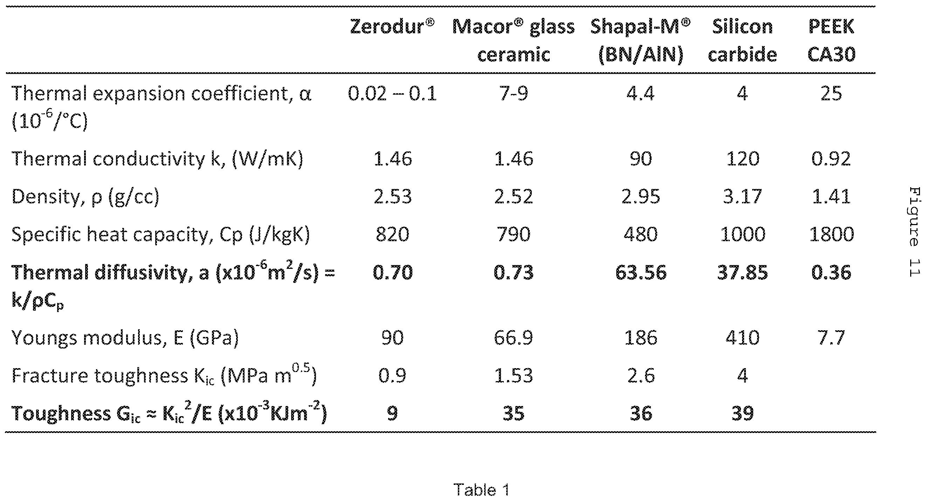

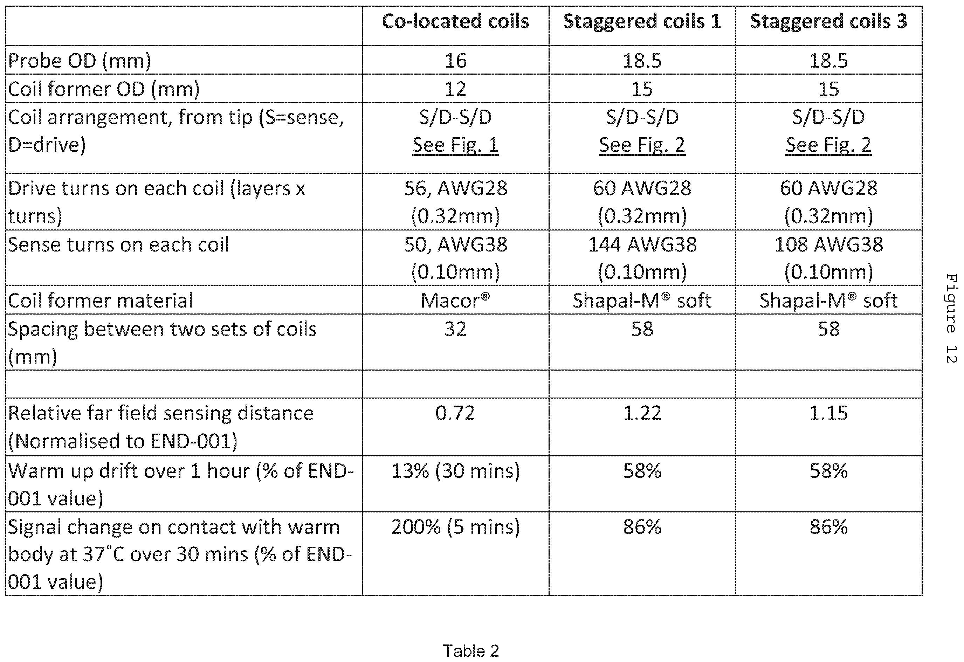

Figures 11 and 12 provide tables 1 and 2 respectively, which are referred to in the description below.

### DESCRIPTION OF AN EMBODIMENT OF THE INVENTION

First, to improve the sensitivity of the coil while reducing its size, it is important to reduce any thermal effects on the operation of the probe, which is highly sensitive to relative change in geometry between the coils, particularly axial movement, and any change in the size of the coils. One of the thermal effects which must be addressed is any asymmetric expansion of the coil arrangement when the end of the probe comes into contact with a warm body, and the coil nearest to the body experiences a higher temperature than the coil further away. Reducing the temperature differential between the coils, while maintaining the low thermal expansion coefficient of the coil former or probe body, permits this sensitivity to be reduced. In other words, it is important to equalize any temperature differential across the coils rapidly so that a thermal gradient cannot be sustained by a thermal input at one end.

When a substance has high thermal diffusivity, heat moves rapidly through the substance because the substance conducts heat quickly relative to its volumetric heat capacity or "thermal bulk." Therefore, a material with high thermal diffusivity is desirable for the coil former (probe core material) in order to equalize the temperature across the coils as rapidly as possible. Further, suitable materials need to be non-magnetic, non-electrically conducting and have a relatively low thermal expansion coefficient. The low thermal expansion coefficient is required to reduce axial and radial expansion which affects the relative positions of the coils. Table 1 shows relevant properties of various materials.

In one construction, the material preferably has a thermal diffusivity of substantially ≥20 x 10⁻⁶ m²/s and a thermal expansion coefficient of substantially < 3 x 10⁻⁵/°C. More preferably, the material has a thermal diffusivity of substantially ≥50 x 10⁻⁶ m²/s and a thermal expansion coefficient of substantially < 5 x 10⁻⁶/°C. Such materials may include:
Glassy ceramics such as borosilicate based machinable ceramics e.g. Macor® (Corning Inc, New York);
Non-glassy ceramics such as aluminum nitride, boron nitride, silicon carbide;
Composite ceramics such as Shapal-M, a composite of boron nitride and aluminum nitride (Tokuyama Corporation, Shunan City, Japan); and
Carbon- and glass-filled composites, for example glass or carbon filled Polyetheretherketone (PEEK).

Further, it is advantageous for the material to have a high stiffness to avoid change in the position of the coils due to mechanical deformation. In one construction, the material has a Young's Modulus of ≥ 40GPa and preferably ≥ 80GPa, and the material has as high a toughness as can be achieved given the other material constraints. For ceramics, in this application, the likely failure mode is energy- (or shock-) induced brittle fracture, for example when the probe is knocked or dropped. In this case, the relevant material property is the toughness G_{ic} ≈ K_{ic}²/E (KJm⁻²) which, along with other probe information, is tabulated in Table 2.

The probe's sensitivity to temperature changes is further reduced by minimizing heat transfer into the probe from outside of the probe. This is achieved by using a high conductivity polymer material for the case, so that the inside surface of the case is closer to an isothermal surface, thus minimizing thermal gradients inside the probe and providing an insulating layer of air or a highly insulating material such as aerogel or a vacuum gap around the probe core, or former, and windings. In addition to the selection of materials, the coil configurations may be selected to reduce thermal effects.

In one embodiment, the probe includes a coil former with two sense coils and two drive coils. The sense and drive coils are arranged in two pairs, each consisting of one sense coil and one drive coil in close proximity to one another. The probe's magnetic sensing performance is maximized by locating one sense and drive coil pair close to the sensing tip of the probe, and locating the second pair of coils an axial distance away from the first pair of coils. The distance is preferably greater than the diameter of the smallest coil, and more preferably greater than the diameter of the largest coil in the pair. By way of example, in one construction, the diameter of the largest coil is 15mm, and in another construction, the diameter is 12mm. In addition, each pair of coils is arranged such that the voltage induced in each of the sense coils by the field generated by the drive coils is approximately equal and opposite. For example, if there are two sense/drive coil pairs: S1, D1 and S2, D2, separated by at least a coil diameter, the current induced in S1 by the combined drive fields from D1 and D2 is equal and opposite to the current induced in S2 by the combined drive fields from D1 and D2. Preferably, the sense coils are arranged as a first order gradiometer in order to minimize the effect of far field sources.

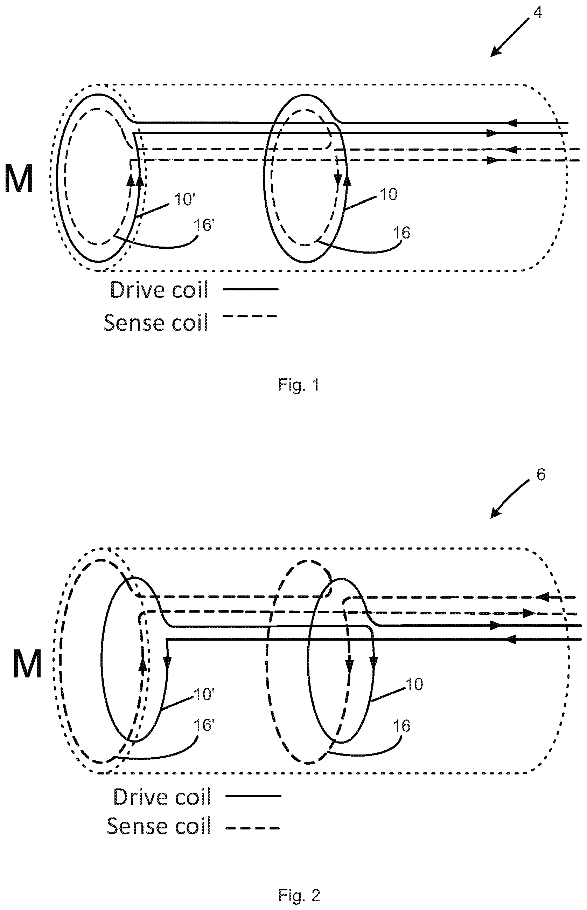

In one construction of the probe 4 (Fig. 1), the coils 10, 10', 16, 16' are co-located such that each drive coil 10, 10' is co-located with a sense coil 16, 16'. Either order of the arrangement of the sense and drive coils can be used. In one construction, the drive coil is wound on top of the sense coil in order to minimize any effect from thermal expansion of the drive coils as the drive coils warm due to ohmic heating. This arrangement is useful where thermal effects are less important than sensitivity at very small diameters (e.g. <15mm). Because this arrangement does not require specific diameters and spacing of the drive and sense coils, it is suitable for use in probes of a very small outer diameter. In one construction, this probe has a diameter of less than 15mm or even less than 10mm in diameter.

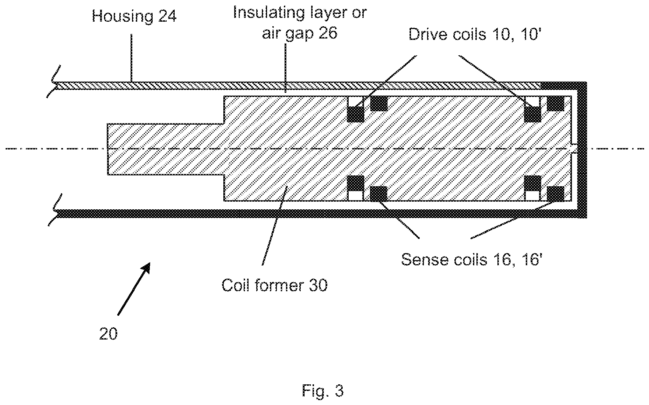

In another exemplary construction of the probe 6 (Fig. 2), drive 10, 10' and sense 16, 16' coils are staggered with the drive coils 10, 10' being separated and the sense coils 16, 16' are divided into two parts. The spacing of the drive coil relative to the sense coil in each pair is chosen such that the effects of the expansion of the larger of the two coils on the mutual inductance of the pair of coils is minimized. This helps further to minimize the effect of thermal expansion. This arrangement provides improved sensing because the sensitivity drops off more slowly with distance. In this arrangement, the sense coil is closest to the sensing end of the probe in order to maximize sensing distance. In this arrangement, from the sensing end, the coil order is SD - SD where D represents a drive coil and S represents a sense coil. Referring to Fig. 3, a cross-section of an exemplary construction of a probe 20 is shown including a housing 24, temperature barrier 26 and a coil former or probe core 30 having circumferential grooves 28 into which the coils 10, 10', 16. 16' are formed.

Other constructions which maintain the relative arrangement of each drive/sense pair also fall within the scope of the disclosure. These examples include different orders and symmetry of the pairs, e.g. SD - DS, DS - DS and DS - SD, as well as varying spacing between the pairs of coils. In each case, the coils are connected such that one of the drive pair of coils or the sense pair of coils forms a gradiometer. The other pair of sense coils or drive coils is wired in series, i.e. with the same sense to form a simple magnetometer. A higher order gradiometer can be used for either set of coils, provided that in each case the order of gradiometer of the drive set of coils differs by one from the order of gradiometer of the sense set of coils. For example, the sense coils may form a second order gradiometer and the drive coils may form a first (or third) order gradiometer.

In one construction, the winding is arranged together with the electronic circuit such that the outside layer of the coil winding is close to ground potential of the electronics. This minimizes the capacitive coupling between the probe and the patient's body which is assumed to be at ground potential.

Further constructions include making the sense coil the larger of the two diameters (rather than the drive coil) in combination with any of the above. If a smaller gauge wire is used for the sense coil (as it carries only a tiny current), then making the sense coil the larger of the two is advantageous because it allows the average diameters of the drive and sense coils to be maximized within the specific arrangement of the sense coil/drive coil pairs. Increasing the diameter and therefore the areas of the coils increases the sensitivity of the magnetic sensor. A larger gauge of wire may be used for the drive coil in order to minimize ohmic heating.

Further, when the diameter of the probe is reduced, the magnetic sensitivity is commensurately reduced by a factor of r⁴ in the near field (drive field drops off with r² and sensing capability with r²) and r⁶ in the far field. Therefore, in order to maintain a similar level of magnetic sensitivity in a smaller diameter probe, the number of turns in the coils, particularly in the sense coils, is increased. However, as a side effect, this also magnifies any noise or drift by the same factor. Thus, drift due to changes in the coil geometry caused by temperature changes should be expected to be magnified.

However, by using the staggered coil arrangement and the Shapal-M coil core, the thermal drift due to warm body contact can be maintained at an acceptable level. For example, with a smaller diameter probe of the staggered coil arrangement, the signal change in response to contact with a warm body at 37°C is about 86% of the equivalent signal change in one of the prior art systems. Because the smaller probe has twice as many coil turns, the signal change should therefore be much greater than this.

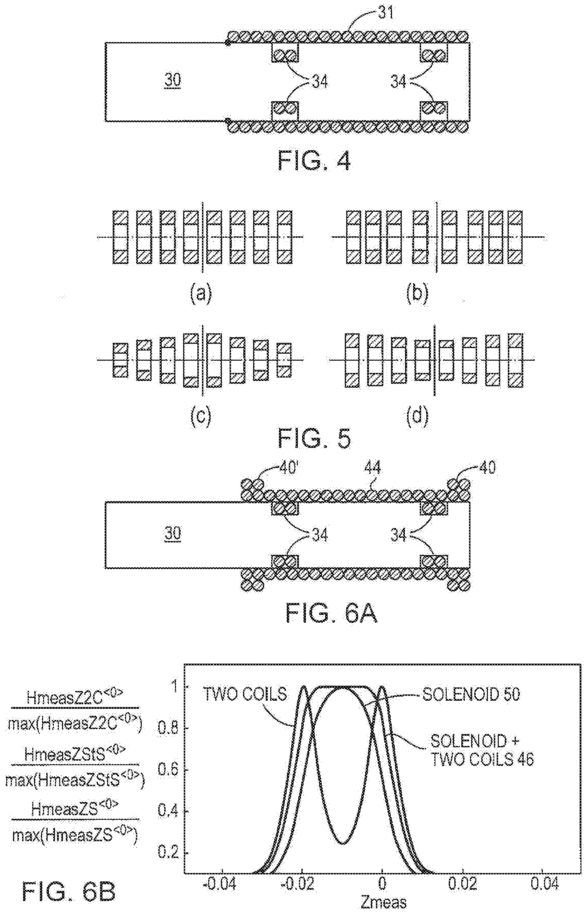

In Figure 4, a solenoid 31 is used as the excitation (drive) coil and two sense coils 34 are positioned inside the solenoid 31. As long as the sense coils 34 are not too close to the end of the solenoid 31 , they will experience a substantially uniform magnetic field from the solenoid 31. The benefit of this arrangement is that small movements relative to the solenoid 31, for example due to thermal expansion, will not disturb the (magnetic) balance of the sense coils 34. Further, the solenoid will also provide an additional heat conduction path to the sense coils and the coil core to help equalize temperature across all the coils. By choosing the wire gauge, the ohmic heating effect of the solenoid can be minimized. The solenoid coil also forms an electrostatic shield for the sense coil.

The more uniform the field is close to the ends of the solenoid, the closer the sense coils can be positioned to the end of the probe, and the better the sensitivity. The uniformity of the field at the ends of the solenoid is optimized by appropriate design of the coils, for example, by varying the spacing, the diameter, both the spacing and diameter, or the shape of the solenoid coils (Fig. 5 (a-d)). Design and manufacturing of such solenoids is known to those skilled in the art.

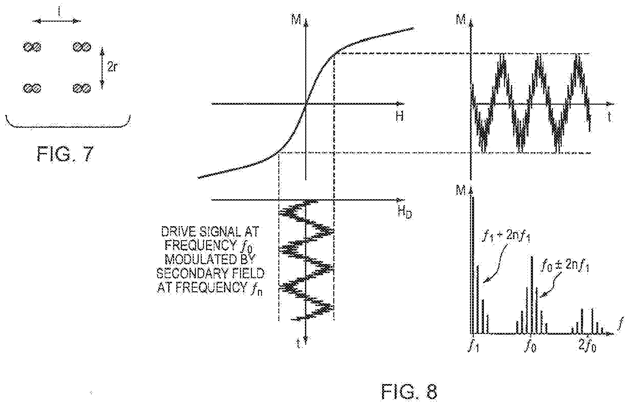

A particular example is shown in Fig. 6a, together with a graph (Fig. 6b), showing the results of modeling the resulting normalized magnetic field strength. By adding two coils 40, 40' at the end of the solenoid 44, the field uniformity along the length can be improved (46) over the field of the solenoid alone (50). A special case of the solenoid drive coil is one in which the drive coil is formed from a Helmholtz pair of coils where the separation between the coils, 1, is substantially equal to the radius of the coils (r) (Fig. 7). The Helmholtz coil is a well-known arrangement that provides a region of constant magnetic field between the two coils, and this region extends further than in any other arrangement of the coils with a different ratio of separation-to-radius.

In addition to manipulating the coils and their structure and locations, embodiments address the issue of distinguishing between the signal from the iron oxide nanoparticles and the signals from other metallic objects. In particular, a second varying magnetic drive field may be generated at a lower frequency than the primary drive field and with comparable or greater magnitude than the primary drive field. The secondary drive field modulates the susceptibility of the magnetic nanoparticles and creates additional frequency components in the spectrum received by the sense coils at frequencies f₀±2nf₁, where f₀ is the primary drive field frequency and f₁ is the secondary drive field frequency and n is a whole number (Fig. 8). The presence of magnetic nanoparticles can be detected by analyzing these additional frequencies that are a result of the mixing of the primary and secondary drive frequencies.

In one embodiment, the amplitude of the side lobe at f₀ ± 2f₁ is measured to detect the presence of magnetic nanoparticles. In an alternative embodiment, the ratio of the amplitude A_{f0±2f1} of the side lobe to the amplitude A_{f0} of the fundamental (A_{f0±2f1}/A_{f0}) is measured. Other embodiments are possible that result in an advantageous combination of frequencies. These components are less sensitive or even insensitive to coil structure and geometric imbalance, temperature effects, and also to eddy currents. Thus, by appropriate signal processing, the undesirable disturbance from coil structure and geometric imbalance, temperature sensitivity, and metallic objects can be reduced or eliminated.

In practice, the geometrical symmetry of construction needed for a perfect electromagnetic balance is very difficult to achieve within the constraints of state-of-the-art and cost-effective construction, and there is usually some level of residual imbalance. Furthermore, temperature fluctuations experienced by the coils, particularly non-uniform changes, for example when the end of the probe (near one coil) comes into contact with a warm body, will create additional dynamic imbalance in the coils as the geometry of the coils varies slightly due to thermal expansion.

These imbalances in the coils manifest themselves as a disturbance signal at the fundamental primary drive frequency f₀ caused by the sense coil detecting a non-cancelled residual direct magnetic signal from the drive coils. However, any signal at a mixed or modulated frequency f₀ ± 2nf₁ resulting from the secondary frequency f₁ is only generated by the magnetic response of the nanoparticles to f₁ and therefore at these frequencies there is no disturbance signal component caused by any coil imbalance. Thus, by extracting these components from the overall signal received by the sense coils, these frequency components can be used to detect the presence of the nanoparticles and distinguish them from disturbance caused by magnetic imbalance of the coils whether caused by imperfections in construction or thermally-induced distortions.

Further, the drive field from the probe will induce eddy currents in any electrically conducting object, e.g. metallic surface that is sufficiently close, and the magnetic field created by the eddy currents may then be picked up by the sense coils as an extraneous signal. However, as with the imbalances described above, there is no frequency mixing with the signals produced by eddy currents. Thus, the mixed or modulated frequencies f₀ ± 2nf₁ do not show any component of fields generated by eddy currents, which means that non-ferromagnetic materials can be distinguished from nanoparticles. Thus, by extracting these components from the overall signal received by the sense coils, these f₁ frequency components can be used to detect the presence of the nanoparticles and distinguish them from non-ferromagnetic materials.

Because the nanoparticles generate side lobes, the existence of side lobes are indicative of nanoparticles and one such measure of the presence of nanoparticles is obtained by measuring the amplitude of the side lobe. The greater the side lobe amplitude, the more nanoparticles are being detected. To adjust for noise in the signals, it is possible to normalize the measurement of the presence of nanoparticles by using the ratio of the amplitude A_{f0±2nf1}, of a side lobe to the amplitude A_{f0} of the fundamental (A_{f0±2nf1} / A_{f0}) where n is a whole number.

An additional benefit of making the system insensitive to eddy currents is that the primary drive frequency can be increased to take advantage of the increased sensitivity of the magnetic nanoparticles at higher frequencies. For example, the frequency could be increased to significantly more than 10kHz; e.g., to 50kHz or 100kHz or 250kHz or more. However, ferromagnetic materials will exhibit both Eddy current and magnetic responses, and have similar non-linear magnetic properties to the nanoparticles. Hence, the magnetic part of the response is not readily distinguishable from the magnetic nanoparticles.

Because the magnetic nanoparticles have a non-linear frequency response, the response of the particles can be distinguished from the diamagnetic effect of the body, which has a linear effect. Therefore, the present invention can also be used to screen out the diamagnetic effect.

The use of a secondary drive field is suitable for use with any of the coil embodiments and, for any given embodiment, the detection of the magnetic field will be much less sensitive to imbalances in the coils. This system does not require a balanced coil arrangement, provided that the fundamental frequency can be filtered out effectively, although practically this may be desirable for other reasons, e.g. to avoid saturating the input electronics.

The frequency of the secondary drive field is chosen to provide sufficient frequency separation from the primary drive frequency band. This may be, for example, in the region of 0.5% to 10% of the primary frequency and preferably in the range of 1% to 5%. For a primary frequency of 100kHz, a secondary frequency of 10Hz to 10kHz and more preferably between 100Hz and 1kHz could be used. For example, in one exemplary construction the primary frequency is 100kHz and the secondary 1kHz. Advantageously, the secondary frequency may be chosen to be a multiple of the power supply frequency, e.g. n x 50 or n x 60Hz, such that the secondary drive can be derived from power supply frequency, but the power supply frequency does not interfere with the sensing. For example, the primary frequency may be 10kHz and the secondary frequency 200Hz. Further, a resonant drive circuit may be used for generating the primary field to maximize the magnetic field strength for a given level of power input. For clinical applications like sentinel lymph node biopsy, a secondary field strength in the region of interest of at least 15 microTesla, and preferably greater than 25 microTesla, is appropriate.

In one embodiment, the secondary field is generated by the handheld probe containing the sensing and primary drive fields, for example, by having a moving permanent magnet. The movement of the magnet can be for example an oscillatory, rotating or vibratory movement. Alternatively, in one embodiment an additional coil is added to the probe and is driven so as to create a time varying magnetic field. In another embodiment, the secondary field is generated away from the probe for example by means of a device placed near to the patient. In another construction, the field is generated in a pad that sits underneath or adjacent to the area of the patient that is being sensed. The field is generated by a coil or multiple coils arranged so as to create a suitable secondary magnetic field. When a single coil is used a coil diameter of at least 200mm is desirable and a field strength of 2.5mT (or H field of 2000 A/m) are desirable at the center of the coil so that there is sufficient secondary field strength in the region of interest where the probe will be used.

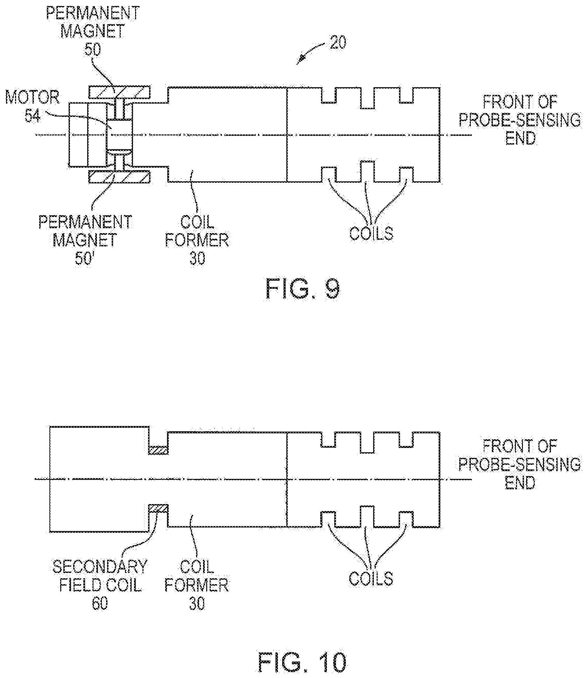

In more detail, and referring to Fig. 9, an alternating secondary field can be generated by rotating a permanent magnet 50, 50' using a motor 54 rotating at the desired frequency. The rotating permanent magnet has the advantage that it is contained within the handheld probe. Such a small motor 54 is capable of spinning a rare earth magnet 50 such that the magnetic polarity of the magnet changes with each rotation. Alternatively, and referring to Fig. 10, the secondary field may be generated electromagnetically by a drive coil 60 located at the rear of the handheld probe driven by a signal generator.

It should be understood that the order of steps or order for performing certain actions is immaterial so long as the present teachings remain operable. Moreover, two or more steps or actions may be conducted simultaneously.

It is to be understood that the figures and descriptions of the invention have been simplified to illustrate elements that are relevant for a clear understanding of the invention, while eliminating, for purposes of clarity, other elements. Those of ordinary skill in the art will recognize, however, that these and other elements may be desirable. However, because such elements are well known in the art, and because they do not facilitate a better understanding of the invention, a discussion of such elements is not provided herein. It should be appreciated that the figures are presented for illustrative purposes and not as construction drawings. Omitted details and modifications or alternative embodiments are within the purview of persons of ordinary skill in the art.
The invention may be embodied in other specific forms without departing from the essential characteristics thereof. The foregoing embodiments are therefore to be considered in all respects illustrative rather than limiting on the invention described herein. Scope of the invention is thus indicated by the appended claims rather than by the foregoing description.

## Claims

1. A magnetic susceptometer for locating tissue in preparation for excision by surgery, by detecting a magnetic marker within the tissue, the magnetic susceptometer comprising a probe, the probe comprising:
a probe core (30) having a first end and a second end, the probe core defining two regions for containing coils of wire, one of the regions being adjacent the first end of the probe core;
two sense coils (16, 16'), one each of the sense coils being located in a respective one of the regions;
two drive coils (10, 10'), one each of the drive coils being located in a respective one of the regions, each drive coil being arranged to generate a primary alternating magnetic drive field; and
a source of a secondary alternating magnetic drive field,
wherein the regions are separated by a distance equal to or greater than the diameter of one of the drive and sense coils;
wherein the magnetic susceptometer is arranged such that the primary alternating magnetic drive field has a frequency, f₀, and the secondary alternating magnetic drive field has a lower frequency, f₁, from that of the primary alternating magnetic drive field;
wherein the magnetic susceptometer is arranged to detect a signal generated by the magnetic marker at a mixing frequency of f₀ ± 2nf₁, where n is a whole number.

2. The magnetic susceptometer of claim 1 wherein the source of the secondary alternating magnetic drive field is a coil (60) located on the probe.

3. The magnetic susceptometer of claim 1 wherein the frequency of the secondary alternating magnetic drive field is less than the frequency of the primary alternating magnetic drive field.

4. The magnetic susceptometer of claim 1 wherein the source of the secondary alternating magnetic drive field is a rotating magnet (50, 50') being driven by a motor (54) located in the probe.

5. The magnetic susceptometer of claim 1 wherein the probe core (30) comprises a material with a thermal diffusivity of substantially ≥ 20 x 10⁻⁶ m²/s and a thermal expansion coefficient of substantially < 3 x 10⁻⁵/°C.

6. The magnetic susceptometer of claim 1 wherein the probe core (30) comprises a material that has a thermal diffusivity of substantially ≥ 50 x 10⁻⁶ m²/s and a thermal expansion coefficient of substantially < 5 x 10⁻⁶/°C.

7. The magnetic susceptometer of claim 1 wherein the frequency of the secondary alternating magnetic drive field is less than or equal to 10% of the frequency of the primary alternating magnetic drive field.

8. The magnetic susceptometer of claim 1 wherein the sense coils (16, 16') and drive coils (10, 10') have different diameters, and the diameter of the sense coils is greater than the diameter of the drive coils.

9. The magnetic susceptometer of claim 1 wherein the probe core (30) comprises a ceramic selected from the group consisting of boron nitride, aluminium nitride, silicon carbide, a ceramic which is a composite of boron nitride and aluminium nitride, and a borosilicate based machinable glassy ceramic.

10. The magnetic susceptometer of any of claims 1 to 9 wherein the probe has a sensing tip at one of the ends of the probe core.

11. A method for locating tissue in preparation for excision by surgery, by detecting a magnetic marker within the tissue comprising:
generating a primary alternating magnetic drive field of frequency f₀;
generating a secondary alternating magnetic drive field of frequency f₁; and
measuring the ratio of the amplitude of a side lobe magnetic field generated by the magnetic marker in response to the primary and secondary alternating magnetic drive fields at a frequency f₀ ± 2nf₁, where n is a whole number > 0, to the amplitude of the primary alternating magnetic drive field.

12. The method of claim 11 wherein the amplitude (A_{f0±2f1}) of the side lobe is measured at a frequency equal to f₀±2f₁.

13. The method of claim 11 or 12 wherein the frequency of the secondary alternating magnetic drive field is less than or equal to 10% of the frequency of the primary alternating magnetic drive field.

14. The method of any of claims 11 to 13 wherein the method is carried out using a magnetic susceptometer comprising a probe,
the probe comprising:
a probe core (30) having a first end and a second end, the probe core defining two regions for containing coils of wire, one of the regions being adjacent the first end of the probe core;
two sense coils (16, 16'), one each of the sense coils being located in a respective one of the regions;
two drive coils (10, 10'), one each of the drive coils being located in a respective one of the regions, the drive coils being arranged to generate the primary alternating magnetic drive field; and
a source of the secondary alternating magnetic drive field,
wherein the regions are separated by a distance equal to or greater than the diameter of one of the drive and sense coils;
wherein the magnetic susceptometer is arranged to use the sense coils to detect a signal generated by the magnetic marker at the frequency of f₀ ± 2nf₁.

## Patentansprüche

1. Magnetisches Suszeptometer zum Lokalisieren von Gewebe in Vorbereitung auf chirurgische Exzision durch Nachweisen eines magnetischen Markers innerhalb des Gewebes, wobei das magnetische Suszeptometer eine Sonde umfasst, wobei die Sonde Folgendes umfasst:
einen Sondenkern (30) mit einem ersten Ende und einem zweiten Ende, wobei der Sondenkern zwei Bereiche zur Aufnahme von Drahtspulen definiert, wobei einer der Bereiche an das erste Ende des Sondenkerns angrenzt;
zwei Abtastspulen (16, 16'), wobei sich jeweils eine der Abtastspulen in jeweils einem der Bereiche befindet;
zwei Treiberspulen (10, 10'), wobei sich jeweils eine der Treiberspulen in jeweils einem der Bereiche befindet und jede Treiberspule angeordnet ist, um ein primäres magnetisches Treibendes Wechselfeld zu erzeugen; und
eine Quelle eines sekundären magnetischen treibenden Wechselfeldes,
wobei die Bereiche durch einen Abstand getrennt sind, der gleich oder größer ist als der Durchmesser von einer der Antriebs- und Abtastspulen;
wobei das magnetische Suszeptometer so angeordnet ist, dass das primäre magnetische treibende Wechselfeld eine Frequenz f₀ aufweist und das sekundäre magnetische treibende Wechselfeld eine niedrigere Frequenz f₁ aufweist als diejenige des primären magnetischen treibenden Wechselfeldes; wobei das magnetische Suszeptometer angeordnet ist, um ein von dem magnetischen Marker erzeugtes Signal bei einer Mischfrequenz von f₀ ± 2nf₁ nachzuweisen, wobei n eine ganze Zahl ist.

2. Magnetisches Suszeptometer nach Anspruch 1, wobei die Quelle des sekundären magnetischen treibenden Wechselfeldes eine auf der Sonde angeordnete Spule (60) ist.

3. Magnetisches Suszeptometer nach Anspruch 1, wobei die Frequenz des sekundären magnetischen treibenden Wechselfeldes geringer ist als die Frequenz des primären magnetischen treibenden Wechselfeldes.

4. Magnetisches Suszeptometer nach Anspruch 1, wobei die Quelle des sekundären magnetischen treibenden Wechselfeldes ein rotierender Magnet (50, 50') ist, der von einem in der Sonde angeordneten Motor (54) angetrieben wird.

5. Magnetisches Suszeptometer nach Anspruch 1, wobei der Sondenkern (30) aus einem Material mit einem thermischen Diffusionsvermögen von im Wesentlichen ≥ 20 x 10⁻⁶ m²/s und einem Wärmeausdehnungskoeffizienten von im wesentlichen < 3 x 10⁻⁵/°C besteht.

6. Magnetisches Suszeptometer nach Anspruch 1, wobei der Sondenkern (30) aus einem Material besteht, das ein thermisches Diffusionsvermögen von im Wesentlichen ≥ 50 x 10⁻⁶ m²/s und einen Wärmeausdehnungskoeffizienten von im Wesentlichen < 5 x 10⁻⁶/°C aufweist.

7. Magnetisches Suszeptometer nach Anspruch 1, wobei die Frequenz des sekundären magnetischen treibenden Wechselfeldes kleiner oder gleich 10% der Frequenz des primären magnetischen treibenden Wechselfeldes ist.

8. Magnetisches Suszeptometer nach Anspruch 1, wobei die Abtastspulen (16, 16') und Treiberspulen (10, 10') unterschiedliche Durchmesser aufweisen und der Durchmesser der Abtastspulen größer ist als der Durchmesser der Treiberspulen.

9. Magnetisches Suszeptometer nach Anspruch 1, wobei der Sondenkern (30) eine Keramik umfasst, die aus der Gruppe ausgewählt ist bestehend aus Bornitrid, Aluminiumnitrid, Siliciumcarbid, einer Keramik, die ein Verbundwerkstoff von Bornitrid und Aluminiumnitrid ist, und einer Borsilicat-basierten zerspanbaren glasartigen Keramik.

10. Magnetisches Suszeptometer nach einem der Ansprüche 1 bis 9, wobei die Sonde eine Sensorspitze an einem der Enden des Sondenkerns aufweist.

11. Verfahren zur Lokalisierung von Gewebe in Vorbereitung auf chirurgische Exzision, durch Nachweisen eines magnetischen Markers innerhalb des Gewebes, umfassend:
Erzeugen eines primären magnetischen treibenden Wechselfeldes der Frequenz f₀;
Erzeugen eines sekundären magnetischen treibenden Wechselfeldes der Frequenz f₁; und
Messen des Verhältnisses der Amplitude eines magnetischen Seitenlappenfeldes, das von dem magnetischen Marker als Reaktion auf die primären und sekundären magnetischen treibenden Wechselfelder bei einer Frequenz f₀ ± 2nf₁ erzeugt wird,
wobei n eine ganze Zahl > 0 ist, zur Amplitude des primären magnetischen treibenden Wechselfeldes.

12. Verfahren nach Anspruch 11, wobei die Amplitude (A_{f0±2f1}) des Seitenlappens bei einer Frequenz gleich f₀ ± 2f₁ gemessen wird.

13. Verfahren nach Anspruch 11 oder 12, wobei die Frequenz des sekundären magnetischen treibenden Wechselfeldes kleiner oder gleich 10% der Frequenz des primären magnetischen treibenden Wechselfeldes ist.

14. Verfahren nach einem der Ansprüche 11 bis 13, wobei das Verfahren unter Verwendung eines magnetischen Suszeptometers, das eine Sonde umfasst, durchgeführt wird,
wobei die Sonde Folgendes umfasst:
einen Sondenkern (30) mit einem ersten Ende und einem zweiten Ende, wobei der Sondenkern zwei Bereiche zur Aufnahme von Drahtspulen definiert, wobei einer der Bereiche an das erste Ende des Sondenkerns angrenzt;
zwei Abtastspulen (16, 16'), wobei sich jeweils eine der Abtastspulen in jeweils einem der Bereiche befindet;
zwei Treiberspulen (10, 10'), wobei sich jeweils eine der Treiberspulen in jeweils einem der Bereiche befindet, wobei die Treiberspulen angeordnet sind, um das primäre magnetische treibende Wechselfeld zu erzeugen; und
eine Quelle des sekundären magnetischen treibenden Wechselfeldes,
wobei die Bereiche durch einen Abstand getrennt sind, der gleich oder größer ist als der Durchmesser einer der Antriebs- und Abtastspulen;
wobei das magnetische Suszeptometer angeordnet ist, um die Abtastspulen zum Nachweisen eines von dem magnetischen Marker erzeugten Signals bei der Frequenz von f₀ ± 2nf₁ zu verwenden.

## Revendications

1. Susceptomètre magnétique pour localiser un tissu lors de la préparation pour l'excision par chirurgie, en détectant un marqueur magnétique à l'intérieur du tissu, le susceptomètre magnétique comprenant une sonde, la sonde comprenant :
un noyau de sonde (30) ayant une première extrémité et une deuxième extrémité, le noyau de sonde définissant deux régions pour contenir des bobines de fil, l'une des régions étant adjacente à la première extrémité du noyau de sonde ;
deux bobines de détection (16, 16'), chacune des bobines de détection étant située dans l'une respective des régions ;
deux bobines d'excitation (10, 10'), chacune des bobines d'excitation étant située dans l'une respective des régions, chaque bobine d'excitation étant conçue pour générer un champ d'excitation magnétique alternatif primaire ; et
une source d'un champ d'excitation magnétique alternatif secondaire,
dans lequel les régions sont séparées par une distance supérieure ou égale au diamètre de l'une des bobines d'excitation et de détection ;
où le susceptomètre magnétique est conçu de sorte que le champ d'excitation magnétique alternatif primaire ait une fréquence, f₀, et le champ d'excitation magnétique alternatif secondaire ait une fréquence, f₁, inférieure à celle du champ d'excitation magnétique alternatif primaire ;
où le susceptomètre magnétique est conçu pour détecter un signal généré par le marqueur magnétique à une fréquence de mélange égale à f₀±2nf₁, où n est un nombre entier.

2. Susceptomètre magnétique de la revendication 1, dans lequel la source du champ d'excitation magnétique alternatif secondaire est une bobine (60) située sur la sonde.

3. Susceptomètre magnétique de la revendication 1, dans lequel la fréquence du champ d'excitation magnétique alternatif secondaire est inférieure à la fréquence du champ d'excitation magnétique alternatif primaire.

4. Susceptomètre magnétique de la revendication 1, dans lequel la source du champ d'excitation magnétique alternatif secondaire est un aimant rotatif (50, 50') qui est entraîné par un moteur (54) situé dans la sonde.

5. Susceptomètre magnétique de la revendication 1, dans lequel le noyau de sonde (30) comprend un matériau ayant une diffusivité thermique essentiellement ≥ 20 x 10⁻⁶ m²/s et un coefficient de dilatation thermique essentiellement < 3 x 10⁻⁵/°C.

6. Susceptomètre magnétique de la revendication 1, dans lequel le noyau de sonde (30) comprend un matériau qui a une diffusivité thermique essentiellement ≥ 50 x 10⁻⁶ m²/s et un coefficient de dilatation thermique essentiellement < 5 x 10⁻⁶/°C.

7. Susceptomètre magnétique de la revendication 1, dans lequel la fréquence du champ d'excitation magnétique alternatif secondaire est inférieure ou égale à 10 % de la fréquence du champ d'excitation magnétique alternatif primaire.

8. Susceptomètre magnétique de la revendication 1, dans lequel les bobines de détection (16, 16') et les bobines d'excitation (10, 10') ont des diamètres différents, et le diamètre des bobines de détection est supérieur au diamètre des bobines d'excitation.

9. Susceptomètre magnétique de la revendication 1, dans lequel le noyau de sonde (30) comprend une céramique choisie dans le groupe consistant en le nitrure de bore, le nitrure d'aluminium, le carbure de silicium, une céramique qui est un composite de nitrure de bore et de nitrure d'aluminium, et une céramique vitreuse usinable à base de borosilicate.

10. Susceptomètre magnétique de l'une des revendications 1 à 9, dans lequel la sonde a une pointe de détection au niveau de l'une des extrémités du noyau de sonde.

11. Procédé pour localiser un tissu lors de la préparation pour l'excision par chirurgie, en détectant un marqueur magnétique à l'intérieur du tissu, comprenant les étapes consistant à :
générer un champ d'excitation magnétique alternatif primaire de fréquence f₀ ;
générer un champ d'excitation magnétique alternatif secondaire de fréquence f₁ ; et
mesurer le rapport de l'amplitude d'un champ magnétique de lobe latéral généré par le marqueur magnétique en réponse aux champs d'excitation magnétiques alternatifs primaire et secondaire à une fréquence f₀±2nf₁, où n est un nombre entier > 0, sur l'amplitude du champ d'excitation magnétique alternatif primaire.

12. Procédé de la revendication 11, dans lequel l'amplitude (A_{f0±2f1}) du lobe latéral est mesurée à une fréquence égale à f₀±2f₁.

13. Procédé de la revendication 11 ou 12, dans lequel la fréquence du champ d'excitation magnétique alternatif secondaire est inférieure ou égale à 10 % de la fréquence du champ d'excitation magnétique alternatif primaire.

14. Procédé de l'une des revendications 11 à 13, où le procédé est réalisé en utilisant un susceptomètre magnétique comprenant une sonde,
la sonde comprenant :
un noyau de sonde (30) ayant une première extrémité et une deuxième extrémité, le noyau de sonde définissant deux régions pour contenir des bobines de fil, l'une des régions étant adjacente à la première extrémité du noyau de sonde ;
deux bobines de détection (16, 16'), chacune des bobines de détection étant située dans l'une respective des régions ;
deux bobines d'excitation (10, 10'), chacune des bobines d'excitation étant située dans l'une respective des régions, les bobines d'excitation étant conçues pour générer le champ d'excitation magnétique alternatif primaire ; et
une source du champ d'excitation magnétique alternatif secondaire,
dans lequel les régions sont séparées par une distance supérieure ou égale au diamètre de l'une des bobines d'excitation et de détection ;
dans lequel le susceptomètre magnétique est conçu pour utiliser les bobines de détection pour détecter un signal généré par le marqueur magnétique à la fréquence f₀±2nf₁.
